# EUROPEAN PATENT APPLICATION

(11) **EP 0 627 435 A1**
(43) Date of publication of application: **07.12.1994**
(21) Application number: 93108752.2
(22) Date of filing: 01.06.1993
(51) Int. Cl.: C07D 475/04

(54) **Method for the industrial preparation of (6S) folic acid derivatives by chromatographic separation**

(71) Applicant: ACS DOBFAR S.p.A., I-20067 Tribiano, Milan (IT)
(72) Inventor: Ambrosini, Leonardo, I-24100 Bergamo (BG) (IT); Sala, Bruno, I-20156 Trezzo d'Adda (MI) (IT)
(74) Representative: Giambrocono, Alfonso, Dr. Ing.

(57) **Abstract**

Method for the industrial preparation of (6S) folic acid derivatives by chromatographically separating a solution of the two (6RS) diastereoisomers on a chromatographic column wherein the separating agent is an albumin in a buffered solution having a pH of about 5.

## Description

The present invention relates to a method for the industrial preparation of (6S) folic acid derivatives by chromatographically separating the diastereoisomeric mixtures thereof on a column, particularly it relates to a method for the industrial preparation of 5-(methyl)-(6S)-tetrahydrofolic acid and 5-(formyl)-(6S)-tetrahydrofolic acid herein referred as "MTHF" and "FTHF". MTHF and FTHF are physiological molecules which together with the less stable 6,10-methylenetetrahydrofolic and tetrahydrofolic acids constitute the in vivo active forms of folic acid, commonly named as folic acid co-factors.

From the therapeutical point of view, both MTHF and FTHF find an application in all of the forms of folate-deficiencies, as general liver protective, and more recently, in antitumor therapies.

MTHF and FTHF and the therapeutically acceptable derivatives thereof commonly used show the following formula (I): 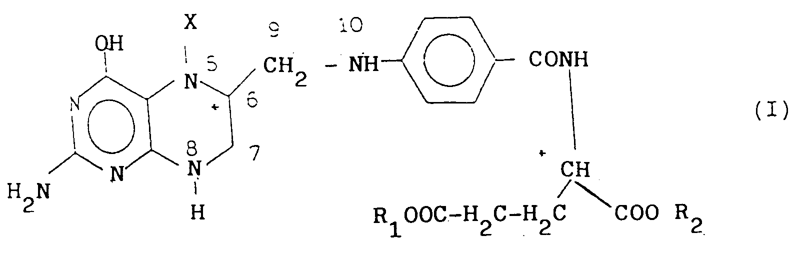
wherein
X= CH₃ or CHO and
R₁ and R₂ are H, NH₄⁺, alkaline and earth-alkaline metals, R₁ and R₂ being equal or different to one another.

The compounds having formula I contain two asymmetric carbon atoms and can therefore exist in four diastereoisomeric forms.

Usually, it is well known that when there exist stereoisomeric pharmaceutical forms, only one of them is active, the other being inactive or even harmful. In the present case, it is well known that the active forms are the (6S) ones.

Some studies have been carried out as to the industrial preparation and/or the isolation of the two diastereoisomers and as to the direct synthesis of the (6S) form.

The stereospecific synthesis of the (6S) form of both MTHF an FTHF, as well as the separation of the two (6RS) diastereoisomers have revealed to be difficult given the structural fragility of the final products.

The syntheses of the stereoisomeric mixtures of MTHF and of FTHF have been described in the GB-A-1.572.138 patent and in the CH-A-496.012 patent respectively.

To separate the (6S) diastereoisomers of the mixtures so obtained, it is possible to transform the stereoisomeric mixture in the respective 5-methyloxy carbonyl derivative or into other derivatives having chiral centres: in such a way, exploiting different solubilities in appropriate solvents, it is possible to separate the two (6R) and (6S) diastereoisomers (JCS, CHEM COMM 470, 1987; EP 0 266 042).

Said method is yet somewhat complicated and the yields are extremely poor.

Other methods of separation are based on multiple crystallizations either of (6R, S) mixtures (EP 0 367 902; WO 88 08,844) or of an intermediate thereof (EP 348,841), always obtaining poor yields.

More recently, the resolution of the stereoisomeric mixtures of both MTHF and FTHF has been obtained (US 5006655) by fractionally crystallizing and aptionally hydrolysing or reducing the 5,10 methylene-(6RS) tetrahydrofolic intermediate, which is hard to be handled and is isolated from a harmful and toxic solvent such as formic acid.

A method for separating the (6S) form is disclosed in PCT/EP88/00341 (W088/08844) wherein a solution of the calcium or magnesium salt of the stereoisomeric mixture is treated with an amine, the cation salt as oxalate precipitating and the desired product being obtained by fractioned crystallization and reconversion to the calcium or magnesium salt.

The yields shown are rather low and the method involves several salifying and crystallizing steps.

In a further resolution process, the racemic mixture, as calcium salt (EP 367,902) is added with a number of organic and inorganic salts, particularly with sodium iodide, thus obtaining a product showing a good purity degree, but once again attaining rather poor yields, surely meaningless from an industrial point of view.

A still further method as to the separation of the isomers of FTHF is described by Choi et al., Analytical Biochem. Vol. 168, pp. 398-404 (1968), according to whom a column of silica gel bound with Bovine Serum Albumin (BSA) is used.

As to the elution, phosphate buffers at a pH comprised between 6,4 and 8,4, preferably 7,4, are used.

The first eluate obtained according to Choi et al contains the (6S) isomer in very low amounts, solely usable for an analytical purpose.

The Choi et al method applied at industrial conditions, would give a rather high stay time of the solution into the column as a result, because of a poor resolution: the industrial productivity would therefore result inadequate because it would be necessary to use columns extremely long to get an acceptable resolution or alternatively subject the solution in the same column to several cycles, which would involve increased possibilities of degradation of the mixture and a very high consumption of the reagents.

Surprisingly it has been found that according to the present invention, following the teachings of Choi et al. but using as to the elution of the (6R,S) mixtures a buffered solution in a very narrow range of pH (between 5.0 and 5.5), the resolution of the (6S) isomer is obtained with industrial yields, cheaply and by a simple procedure.

Indeed, if one uses two industrial columns charged with silica gel bound with an albumin to resolve the (6RS) mixture according to the method of Choi et al. and respectively to the present invention, a yield of the (6S) isomer lower than 5% and not lower tha 20% is respectively attained.

Besides to the above mentioned methods of separating the (6S) isomers from the (6R,S) mixtures, efforts have been made to directly synthesize the (6S) isomers.

According to the EP-A-356,984 patent, the synthesis of the (6S) isomer has been accomplished by a method based on the stereospecific hydrogenation of the 5-6 double bond of 7,8-dihydrofolic acid.

When said hydrogenation is carried out with dihydrofolate reductase in the presence of NADP, a good conversion in obtained: said method is however extremely complicated and not at all usable from an industrial point of view.

Other kinds of stereospecific hydrogenations which provide the use of appropriate catalysts have been attempted but always with poor results (TETRAHEDRON 42, 117, 1986).

Finally, no cheap method exists nowadays which is usable on an industrial scale to attain the (6S) isomers of MTHF and FTHF. As to the practical therapeutical use, (6S) MTHF and (6S) FTHF are not used per se but as salts, usually calcium salts.

Such a transformation is carried out according to well known methods, for instance according to the methods disclosed in the US-A-2,688,018 patent and by Weast in "Handbook of Chemistry and Physics" (57^{th} ed:, pag. B-100).

A mixture of (6RS) MTHF or (6RS) FTHF is separated into the respective (6S) isomers by a method according to which in a chromatographic column an aqueous solution of an albumin is charged, then a washing is carried out with a first buffered solution, an aqueous solution of the diastereosimeric mixture of the derivative of formula (I) is charged, then eluting with a second buffered solution obtaining the (6S) diastereoisomer as first eluate, characterized in that the concentration of the albumin in the solution thereof is comprised between 0,1% and 10% by weight and that the solution of the derivatives has a concentration between 0,1% amd 10% by weight, the pH of said buffered solutions being comprised between 4,8 and 5,8.

Preferably the column is washed with a buffered solution at pH 7 and then again at pH 5 before that the diastereoisomeric mixture of the derivative of formula (I) is charged.

Still preferably, the aqueous solutions of the albumin and of the diastereoisomeric mixture are buffered at a pH comprised between 4.8 and 5.8.

Still further preferably, the silica gel has a granulometry comprised between 25 and 60 micron, still better between 35 and 45 micron.

Advantageously said buffered solutions are phosphate buffers having a concentration comprised between 0,05M and 0,15M, the concentration of said albumin in the aqueous solution thereof is about 1% by weight and that one of the diastereoisomeric mixture in its solution is about 5% by weight, while the albumin is selected from the group comprising pork, bovine, ovine, rabbit, chicken and egg albumin.

Obviously, the buffered solutions used in the various phases of the method according to the present invention can be equal or different between themselves. As buffered solutions phtalate, phosphate, acetate, etc. can be used, preferably phosphate.

According to a preferred method, a 0,05M phosphate buffer is used to elute the chromatographic column wherein a solution of (6R,S)-methyl-tetrahydrofolic acid, calcium salt has been previously charged (a 0,15M phosphate buffer in the case of (6R,S) formyl-tetrahydrofolic acid, calcium salt).

Finally, according to the present invention it has been possible to carry the productivity of the separating method to industrial levels, attaining eluting times extremely shortened.

Now, the present invention will be described in detail with reference to the preferred embodiments. However, it should be understood that the present invention is by no means restricted to these specific embodiments.

The yields set out in the following examples have been expressed as ponderal values whereto it corresponds a double optical yield.

### EXAMPLE 1

In an industrial column having a diameter of 0,90m and a height of 6.0m, there is charged the silica gel (particles diameter 35/45 micron), suspended in a 0,15M buffered aqueous solution at pH 5 containing mercaptoethanol (0,1% by weight) till almost complete filling (height of 5,5 m equal to a volume of about 3600 g of silica gel).

The preparation of the chiral substrate is carried out in the following way: a solution of egg serum albumin (1% in 0,15M phosphate buffer, pH 5,0) is charged into the column, following the absorption in UV at 280 mm on the eluted liquid. The amount of the albumin absorbed on the silica gel is equal to 200/400 kg. Elution is carried out with 10000 l of 0,15M phosphate buffer at pH 5,0 then with 10000 1 of 0,15M phosphate buffer at pH 7 and finally, with 10000 l of 0,15M phosphate buffer at pH 5,0. A 5% solution of calcium 5-formyl-(6R,S)tetrahydrofolate equivalent to 5 kg having a flow rate of 200/400 l/h is then added eluting with a 0,15M phosphate buffer at pH 5,0. Fractions of 500 l are collected.

The (6S) fractions are eluted before of the (6R) fractions. The elution of the fractions can be line - controlled by a polarimeter equipped with flow cell and then accurately dosed by HPLC. The fractions sufficiently pure are isolated as to the required purposes. The pure fractions are collected and salified with konwn methods.
Yield: 1,9 kg (28%) of calcium (6S) folinate
Titre: HPLC >98%
Optical Purity >97%

### EXAMPLE 2

In the present examples the teachings of Choi et al. have been reproduced on an industrial scale.

Using both the same kind of column and the same method of the preparation of the stationary phase, the method is carried out as in the Example 1.

The elution is carried out with 10000 l of 0,15M phosphate buffer at pH 7. A 5% solution of Ca (6R,S)-folinate equivalent to about 5 kg is added to the column, flow rate 200/400 l/h. The elution is carried out with phosphate buffer at pH 7. Fractions of 500 l are collected.

The resolution is unsatisfactory; the purification cycle is repeated four times in order to attempt to obtain a partial resolution.

The text is discarded for lacking resolution.

### EXAMPLE 3

The same kind of column and the same method of the preparation of the stationary phase are used as in the Example 1, but Pork Serum Albumin (PSA) is herein used.
Yield: 1,5 kg (30%) of Ca(6S) folinate
HPLC titre: >98%
Optical purity: >97%

### EXAMPLE 4

It has been used the same kind of column as in the Example 1, but degassed water has been herein used as to the preparation of the stationary phase and as to the next phases of absorption and elution.

10000 l of 0,05M phosphate buffer at pH 5,2 are used for the elution, a 5% solution of (6S)-methyltetrahydrofolic acid equivalent to 10 kg with a flow rate of 200/400 l/h is therewith added, elution being again carried out with 0,05M phosphate buffer at pH 5,2 and 500 l fractions are then collected.

The (6S) fractions are eluted before of the (6R) fractions. The fractions sufficiently pure are isolated as to the required purposes, collected and processed with known methods.
Yield: 3,9 kg (39%) of calcium methyltetrahydro
folate (6S)
HPLC Titre: >97%
Optical purity: >97%

## Claims

1. Method for the industrial preparation of the (6S) isomers by chromatographically separating on a chromatograohic column (6R,S) diastereoisomeric mixtures of folic acid derivatives of formula (I): 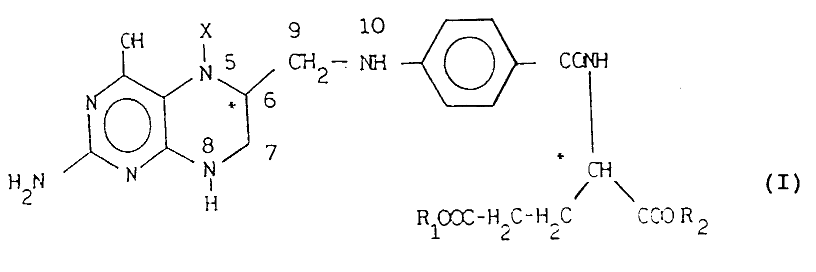 wherein
X= CH₃ or CHO and
R₁ and R₂ are H, NH₄⁺, alkaline and earth-alkaline metals, R₁ and R₂ being equal or different to one another,
wherein in a chromatographic column an aqueous solution of an albumin is charged, then a washing is carried out with a first buffered solution, an aqueous solution of the diastereoisomeric mixture of the derivative of formula (I) is charged, then eluting with a second buffered solution and obtaining the (6S) diastereoisomer as first eluate, characterized in that the concentration of the albumin in the solution thereof is comprised between 0,1% and 10% and that the solution of the derivative has a concentration between 0,1% and 10%, the pH of said buffered solutions being comprised between 4,8 and 5,8.

2. Method according to claim 1, characterized in that the column is washed with a buffered solution at pH 7 and then again at pH 5 before charging the diastereoisomeric mixture of the derivative of formula I.

3. Method according to claims 1 and 2, characterized in that said buffered solutions are phosphate buffers having a concentration comprised between 0,05M and 0,15M.

4. Method according to claims 1 to 3 characterized in that said chromatographic column is charged with a silica gel having a granulometry comprised between 25 and 60 micron.

5. Method according to claim 4, characterized in that the granulometry of the silica gel is comprised between 35 and 45 micron.

6. Method according to claims 1 to 5, characterized in that the concentration of said albumin in the aqueous solution thereof is about 1% by weight and that one of said diastereoisomeric mixture is about 5% by weight.

7. Method according to claims 1 to 6, characterized in that said albumin is selected from the group comprising pork, bovine, ovine, rabbit, chicken and egg albumin.

8. Method according to claims 1 to 7, characterized in that said albumin aqueous solution is buffered at a pH comprised between 4,8 and 5,8.

9. Method according to claims 1 to 7, characterized in that said aqueous solution of said diastereoisomeric mixture is buffered at a pH comprised between 4,8 and 5,8.
